# EUROPÄISCHE PATENTSCHRIFT

(11) **EP 2 111 552 B1**
(45) Veröffentlichungstag und Bekanntmachung des Hinweises auf die Patenterteilung: **04.04.2012**
(21) Anmeldenummer: 07856143.8
(22) Anmeldetag: 20.12.2007
(51) Int. Cl.: G01N 33/68, G01N 33/50

(54) **DIAGNOSE UND RISIKOSTRATIFIZIERUNG MITTELS DEM NEUEN MARKER CT-PROADM**
DIAGNOSIS AND RISK STRATIFICATION BY MEANS OF THE NOVEL MARKER CT-PROADM
DIAGNOSTIC ET STRATIFICATION DES RISQUES AU MOYEN DU NOUVEAU MARQUEUR CT-PROADM

(30) Priorität: 20.12.2006 DE 102006060112
(43) Veröffentlichungstag der Anmeldung: 28.10.2009
(73) Patentinhaber: B.R.A.H.M.S GmbH, 16761 Hennigsdorf (DE)
(72) Erfinder: BERGMANN, Andreas, 12351 Berlin (DE); STRUCK, Joachim, 13465 Berlin (DE)
(74) Vertreter: Simandi, Claus
(86) Internationale Anmeldenummer: PCT/DE2007/002300
(87) Internationale Veröffentlichungsnummer: WO 2008/074315

(56) Entgegenhaltungen:
- JP-A- 2007 316 009
- STRUCK J ET AL: "Identification of an Adrenomedullin precursor fragment in plasma of sepsis patients" PEPTIDES, ELSEVIER, AMSTERDAM, Bd. 25, Nr. 8, 1. August 2004 (2004-08-01), Seiten 1369-1372, XP004551479 ISSN: 0196-9781
- GUMUSEL BULENT ET AL: "Adrenotensin: An adrenomedullin gene product contracts pulmonary blood vessels" PEPTIDES (TARRYTOWN), Bd. 17, Nr. 3, 1996, Seiten 461-465, XP002484481 ISSN: 0196-9781
- ZHOU LAN ET AL: "Vasoactive effects of adrenotensin and its interactions with adrenomedullin" CHINESE MEDICAL JOURNAL (ENGLISH EDITION), Bd. 113, Nr. 3, März 2000 (2000-03), Seiten 269-271, XP002484524 ISSN: 0366-6999

## Beschreibung

Die Erfindung betrifft einen neuen diagnostischen Marker CT-proADM (C-terminales Fragment des preproADM, SEQ ID No. 1) zur Diagnose und / oder Risikostratifizierung von Krankheiten. Ferner ein Verfahren zur Diagnose und / oder Risikostratifizierung von Erkrankungen, insbesondere Herz-Kreislauferkrankungen, Herzinsuffizienz, Infektionen und /oder Entzündungen von Lunge und Atemwege, wobei eine Bestimmung des Markers CT-proADM (SEQ ID No. 1) oder enthaltend in einer Markerkombination (Panel, Cluster) an einem zu untersuchenden Patienten durchgeführt wird. Weiterhin betrifft die Erfindung eine diagnostische Vorrichtung und ein Kit zur Durchführung des Verfahrens.

Im Stand der Technik ist die proAdrenomedullin (proADM)-und Adrenomedullin-Bestimmung in der Diagnose beschrieben (EP0622458B1, Lewis LK, Smith MW, Yandle TG, Richards AM, Nicholls MG. Adrenomedullin(1-52) measured in human plasma by radioimmunoassay: plasma concentration, adsorption, and storage. Clin Chem 1998;44:571-7; Ueda S, Nishio K, Minamino N, Kubo A, Akai Y, Kangawa K, et al. Increased plasma levels of adrenomedullin in patients with systemic inflammatory response syndrome. Am J Respir Crit Care Med 1999;160:132-6; Kobayashi K, Kitamura K, Etoh T, Nagatomo Y, Takenaga M, Ishikawa T, et al. Increased plasma adrenomedullin levels in chronic congestive heart failure. Am Heart J 1996;131:994-8; Kobayashi K, Kitamura K, Hirayama N, Date H, Kashiwagi T, Ikushima I, et al. Increased plasma adrenomedullin in acute myocardial infarction. Am Heart J 1996;131:676-80.), insbesondere zwecks Diagnose von Sepsis (EP1121600B1). N-terminale Fragmente von (pre)proAdrenomedullin zur Diagnose sind ebenfalls in EP0622458B1 beschrieben, wie PAMP (Hashida S, Kitamura K, Nagatomo Y, Shibata Y, Imamura T, Yamada K, et al. Development of an ultrasensitive enzyme immunoassay for human proadrenomedullin N-terminal 20 peptide and direct measurement of two molecular forms of PAMP in plasma from healthy subjects and patients with cardiovascular disease. Clin Biochem 2004; 37: 14-21).
Zudem ist ein weiteres Fragment des proAdrenomedullins - nämlich das so genannte midregionale proAdrenomedullin (MRproADM) in EP1488209B1 zu diagnostischen Zwecken offenbart (Struck J, Tao C, Morgenthaler NG, Bergmann A. Identification of an Adrenomedullin precursor fragment in plasma of sepsis patients. Peptides 2004; 25: 1369-72; Morgenthaler NG, Struck J, Alonso C, Bergmann A. Measurement of midregional proadrenomedullin in plasma with an immunoluminometric assay. Clin Chem 2005;51:1823-9; Christ-Crain M, Morgenthaler NG, Stolz D, Muller C, Bingisser R, Harbarth S, et al. Proadrenomedullin to predict severity and outcome in communityacquired pneumonia [ISRCTN04176397]. Crit Care 2006;10:R96; Christ-Crain M, Morgenthaler NG, Struck J, Harbarth S, Bergmann A, Muller B. Mid-regional pro-adrenomedullin as a prognostic marker in sepsis: an observational study. Crit Care 2005; 9: R816-24).

Es besteht jedoch ein hohes Bedürfnis für Erkrankungen, insbesondere Herz-Kreislauferkrankungen, Herzinsuffizienz, Infektionen und / oder Entzündungen von Lunge und Atemwege eine sichere Diagnose zu stellen oder eine (Risiko)Stratifizierung vorzunehmen, insbesondere hinsichtlich weiterer klinischer Entscheidungen und insbesondere hinsichtlich des Schweregrades von Erkrankungen, insbesondere bei Herz-Kreislauferkrankungen, Herzinsuffizienz, Infektionen und / oder Entzündungen von Lunge und Atemwege.

Es ist eine Aufgabe der vorliegenden Erfindung einen neuen Marker bereitzustellen.
Eine weitere Aufgabe der vorliegenden Erfindung besteht darin, ein verbessertes Verfahren zur Diagnose und / oder Risikostratifizierung von Erkrankungen, insbesondere Herz-Kreislauferkrankungen, Herzinsuffizienz, Infektionen und /oder Entzündungen von Lunge und Atemwege bereitzustellen.

Die Aufgabe wird zum einen durch die Bereitstellung des diagnostischen Marker CT-proADM (C-terminales proAdrenomedullin, SEQ ID No. 1) gelöst zum anderen durch ein Verfahren zur in-vitro Diagnose und / oder Risikostratifizierung von Erkrankungen gelöst, wobei eine Bestimmung des Markers CT-proADM (SEQ ID No. 1) oder ein Teilpeptid oder Fragment davon oder enthaltend in einer Markerkombination (Panel, Cluster) an einem zu untersuchenden Patienten durchgeführt wird (nachstehend erfindungsgemäßes Verfahren).

Der Begriff "Risikostratifizierung" umfasst erfindungsgemäß das Auffinden von erkrankten Patienten mit der schlechteren Prognose, zwecks intensiverer Diagnostik und (Folge-) Therapie/Behandlung einer Erkrankung mit dem Ziel einen möglichst günstigen Verlauf der Erkrankung herbeizuführen.

Besonders vorteilhaft kann daher mittels des erfindungsgemäßen Verfahrens eine sichere Diagnose und / oder Risikostratifizierung erfolgen. Das erfindungsgemäße Verfahren ermöglicht klinische Entscheidungen, die zu einer schnelleren Diagnose führen. Solche klinische Entscheidungen umfassen ebenfalls weiterführende Behandlung mittels Arzneimitteln zur Behandlung oder Therapie von Erkrankungen.

Daher betrifft die Erfindung ebenfalls ein in vitro Verfahren zur Risikostratifizierung von Patienten, insbesondere zur Stratifizierung von Patienten für klinische Entscheidungen, vorzugsweise in der zeitlich kritischen Intensivmedizin oder Notfallmedizin und zur Hospitalisierung von Patienten.

In einer weiteren bevorzugten Ausführungsform des erfindungsgemäßen Verfahrens erfolgt die Diagnose und / oder Risikostratifizierung zur Prognose, zur differentialdiagnostischen Früherkennung und Erkennung, zur Beurteilung des Schweregrades und zur therapiebegleitenden Verlaufsbeurteilung von Erkrankungen.

In einer weiteren Ausführungsform des erfindungsgemäßen Verfahrens wird dem zu untersuchenden Patienten Körperflüssigkeit oder Körpergewebe, vorzugsweise Blut entnommen, wahlweise Vollblut, Serum oder erhältliches Plasma und die Diagnose erfolgt *in vitro*/*ex vivo*, d.h. außerhalb des menschlichen oder tierischen Körpers. Aufgrund der Bestimmung des Markers CT-proADM (SEQ ID No. 1) und seiner vorhandenen Menge in mindestens einer Patientenprobe kann die Diagnose und / oder Risikostratifizierung erfolgen.

Im Rahmen dieser Erfindung wird unter "Erkrankungen" Krankheiten des Menschen oder Tier, insbesondere Säugetier, verstanden. Solche Erkrankungen, insbesondere humane Erkrankungen, sind im Pschyrembel, De Gruyter, Berlin 2004 beschrieben.

Besonders vorteilhaft können jedoch im Rahmen dieser Erfindung Herz-Kreislauferkrankungen, Herzinsuffizienz, Infektionen und / oder Entzündungen von Lunge und Atemwege diagnostiziert und / oder stratifiziert werden.

Der Begriff "Herz-Kreislauferkrankungen" umfasst erfindungsgemäß sämtliche Erkrankungen des Herzens und des Blutkreislaufes, insbesondere solche Indikationen, wie Bluthochdruck, koronare Herzerkrankungen, insbesondere akutes Koronarsyndrom, (akuter) Myokardinfarkt, Angina Pectoris.

Der Begriff "akutes Koronarsyndrom" umfasst verschiedene Phasen der koronaren Herzerkrankung, die unmittelbar lebensbedrohlich sind. Dies betrifft insbesondere die Notfallmedizin, und zwar einen akuten Myokardinfarkt und /oder Angina pectoris sowie einen plötzlichen Herztod. Neben dem akuten Myokardinfarkt, der nach WHO-Kriterien (WHO (1979): Nomenclature and criteria for diagnosis of ischemic heart disease. Report of the Joint International Society and Federation of Cardiology/World Health Organization task force on standardization of clinical nomenclature, Circulation 59 (3): 607-609) definiert ist als akutes, länger als 20 Minuten andauerndes Brustschmerzereignis, verbunden mit ST-Streckenhebungen und/oder einer Erhöhung myokardialer Enzyme, wurde der Begriff der instabilen Angina pectoris (AP) geprägt, der erfindungsgemäß unter "akutes Koronarsyndrom" zu lesen ist (Hamm CW: Leitlinien: Akutes Koronarsyndrom (ACS) - Teil 1: ACS ohne persistierende ST-Hebung. Z Kardiol (2004) 93:72-90).

Im Rahmen dieser Erfindung wird unter "Herzinsuffizienz" ein akutes oder chronisches Unvermögen des Herzens, das Gewebe mit ausreichend Blut und infolge dessen genügend Sauerstoff zu versorgen, um den Gewebestoffwechsel in Ruhe oder unter Belastung sicherzustellen. Klinisch liegt eine Herzinsuffizienz vor, wenn typische Symptome (Dyspnoe, Müdigkeit, Flüssigkeitsretention) bestehen, deren ursächlich eine kardiale Funktionsstörung im Sinne einer systolischen oder diastolischen Funktionsstörung zugrunde liegt. Ebenfalls die chronische Herzinsuffizienz (CHF) ist erfindungsgemäß umfasst(Kardiologie compact, herausgegeben von Chrisian Mewis, Reimer Riessen und Ioakim Spyridopoulos, 2. unveränderte Auflage, Thieme 2006). Ursachen einer Herzinsuffizienz können sein: Herzklappenfehler (z. B. als Spätfolge des rheumatischen Fiebers), Myokarditis (Herzmuskelentzündung), Herzrhythmusstörungen, Herzinfarkt neben zu hohem Blutdruck (Hypertonie) und/oder Arteriosklerose (Verkalkung) der Herzkranzgefäße (koronare Herzkrankheit). Weiterhin erfindungsgemäß umfasst sind hypertensive Herzkrankheit mit (kongestiver) Herzinsuffizienz, Hypertensive Herz- und Nierenkrankheit mit (kongestiver) Herzinsuffizienz, Primäre Rechtsherzinsuffizienz, Sekundäre Rechtsherzinsuffizienz, Linksherzinsuffizienz ohne Beschwerden (NYHA-Stadium I), Linksherzinsuffizienz mit Beschwerden bei stärkerer Belastung (NYHA-Stadium II), Linksherzinsuffizienz mit Beschwerden bei leichterer Belastung (NYHA-Stadium III), Linksherzinsuffizienz mit Beschwerden in Ruhe (NYHA-Stadium IV) und kardiogener Schock.

Im Rahmen dieser Erfindung wird unter dem Begriff "Infektionen der Lunge und Atemwege" insbesondere solche Infektionen verstanden, die durch Bakterien, Viren, Pilze oder Parasiten verursacht werden, z.B. solche Indikationen wie tiefe Atemwegsinfektion (LRTI: Lower respiratory tract infections), Bronchitis, Pneumonie, Sarkoidose, Bronchiektasen, Nichtkardiales Lungenödem.
Erfindungsgemäß bevorzugt sind zudem tiefe Atemwegsinfektion (LRTI: Lower respiratory tract infections), Bronchitis, putride Bronchitis, Pneumonie. Ganz besonders bevorzugt ist Pneumonie, insbesondere die ambulant erworbene Pneumonie (CAP: community associated pneumonie), tiefe Atemwegsinfektion (LRTI: Lower respiratory tract infections).

Im Rahmen dieser Erfindung wird unter Pneumonie (Lungenentzündung) eine akute oder chronische Entzündung des Lungengewebes verstanden und dessen Infektion verursacht durch Bakterien, Viren oder Pilze, selten auch toxisch durch Inhalation giftiger Stoffe oder immunologisch. Für den Kliniker ist die Pneumonie eine Konstellation verschiedener Symptome (Fieber oder Hypothermie, Schüttelfrost, Husten, pleuritischer Thoraxschmerz, gesteigerte Sputumproduktion, erhöhte Atemfrequenz, Klopfschalldämpfung, Bronchialatmen, ohrnahe Rasselgeräusche, Pleurareiben) in Kombination mit mindestens einem auf dem Thorax-Röntgenbild erkennbaren Infiltrat (Harrisons Innere Medizin, herausgegeben von Manfred Dietel, Norbert Suttorp und Martin Zeitz, ABW Wissenschaftsverlag 2005).

Im Rahmen dieser Erfindung wird unter dem Begriff "Entzündungserkrankungen der Lunge und Atemwege" bzw. "inflammatorische Erkrankungen der Lunge und Atemwege" solche Indikationen verstanden, wie Interstitielle Lungenerkrankungen und Lungenfibrosen, Chronisch obstruktive Lungenerkrankungen (COPD), insbesondere COPD Infekt-Exazerbationen, Asthma bronchiale, insbesondere Infekt-Exazerbationen bei Asthma bronchiale, Bronchialkarzinom.

COPD bezeichnet erfindungsgemäß eine Gruppe von chronischen Krankheiten, die durch Husten, vermehrten Auswurf und Atemnot bei Belastung gekennzeichnet sind. In erster Linie sind die chronisch-obstruktive Bronchitis und das Lungenemphysem zu nennen. Beide Krankheitsbilder sind dadurch gekennzeichnet, dass vor allem die Ausatmung (Exspiration) behindert ist. Eine umgangssprachliche Bezeichnung für das Hauptsymptom der COPD ist zudem "Raucherhusten". Die Erfindung ist besonders vorteilhaft bei akuten Exazerbationen.

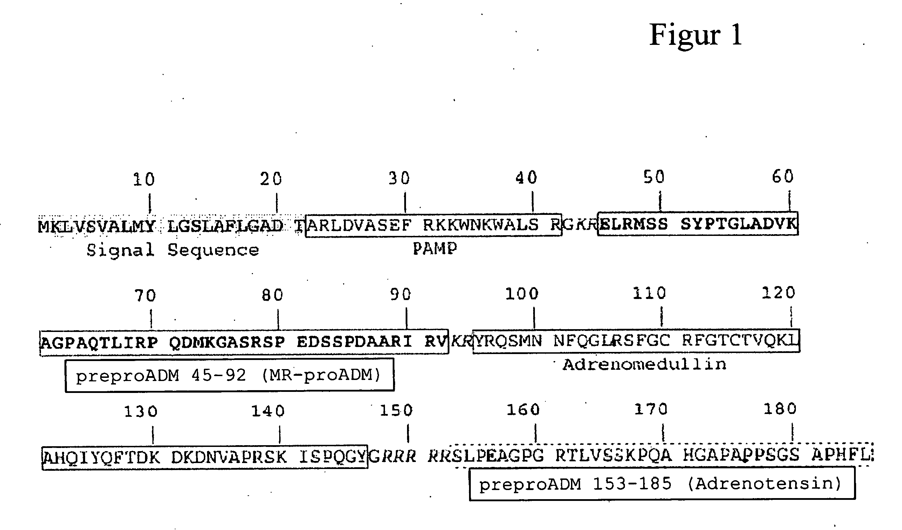

Im Rahmen dieser Erfindung wird unter "CT-proADM" ein freies humanes Protein oder Polypeptid bestehend aus 33 Aminosäuren mit der Aminosäuresequenz - SEQ ID No. 1: SLPEAGPGRTLVSSKPQAHGAPAPPSGSAPHFL - verstanden, bzw. ein Fragment mit einer Aminosäuresequenz von 153-185 (Position 153 ist Ser, Position 185 ist Leu) der SEQ ID No. 2 (Figur 1) des preproAdrenomedullins (Kitamura K, Sakata J, Kangawa K, Kojima M, Matsuo H, Eto T. Cloning and characterization of cDNA encoding a precursor for human adrenomedullin Biochem Biophys Res Commun 1993;194:720-725). Solche Fragmente können zum Beispiel die Aminosäurensequenzen 1-15 der SEQ ID No. 1 bzw. 153-167 des preproAdrenomedullins (Figur 1) oder 19-33 der SEQ ID No. 1 bzw. 171-185 des preproAdrenomedullins (Figur 1) sein (siehe Beispiele). CT-proADM wird aufgrund der vasokonstriktiven Wirkung ebenfalls als Adrenotensin bezeichnet (Gumusel B, Chang JK, Hyman A, Lippton H. Adrenotensin: an ADM gene product with the opposite effects of ADM. Life Sci 1995;57:PL87-90).

Ferner kann das erfindungsgemäße "CT-proADM" Modifikationen aufweisen, wie Glykolisierung, Lip(o)idisierung oder Derivatisierungen.

In einer weiteren Ausführungsform kann die Bestimmung von "CT-proADM" (SEQ ID No. 1) zusätzlich mit weiteren Markern erfolgen, wobei das "CT-proADM" in einer Markerkombination (Panel, Cluster) enthalten ist, und zwar vorzugsweise solche, die bereits auf eine Erkrankung hinweisen. Bevorzugt sind jedoch solche Marker, die wiederum auf die im Rahmen dieser Erfindungen bevorzugten Indikationen/Erkrankungen hinweisen und einen synergetischen Effekt bewirken können.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker aus mindestens einem Marker aus der Gruppe Kreatinkinase, Myeloperoxidase, Copeptin, Myoglobin, natriuretisches Protein, insbesondere ANP (bzw. ANF), proANP, NT-proANP, BNP, proBNP, NT-proBNP oder jeweils eine Teilsequenz davon, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin (proEnd), pro-Leptin, pro-Neuropeptid-Y, pro-Somatostatin, pro- Neuropeptid-YY, pro-Opiomelanocortin, Copeptin oder jeweils eine Teilsequenz davon.
Der ischämische Marker kann aus mindestens einem Marker aus der Gruppe Troponin I und T, CK-MB ausgewählt sein. Darüber hinaus kann der neurohormonale Marker mindestens ein natriuretisches Protein sein, insbesondere ANP (bzw. ANF), proANP, NT-proANP, BNP, proBNP, NT-proBNP oder jeweils eine Teilsequenz davon.
Besonders bevorzugt sind Markerkombinationen von CT-proADM mit einem Prohormon, insbesondere proBNP, NT-proBNP.

In einer weiteren Ausführungsform der Erfindung kann das erfindungsgemäße Verfahren mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen an einem Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), sei es in Einzel- oder Multiparameterbestimmung.

Ferner betrifft die Erfindung die Verwendung von CT-proADM (SEQ ID No. 1) zur in-vitro Diagnose und / oder Risikostratifizierung von Erkrankungen, insbesondere von Herz-Kreislauferkrankungen, Herzinsuffizienz, Infektionen und / oder Entzündungen von Lunge und Atemwege.

In einer weiteren Ausführungsform betrifft die Erfindung die Verwendung von CT-proADM (SEQ ID No. 1) davon zur in vitro Kardialdiagnostik.

Ferner betrifft die Erfindung die Verwendung von CT-proADM (SEQ ID No. 1) davon oder enthaltend in einer Markerkombination (Panel, Cluster) zur in-vitro Diagnose und / oder Risikostratifizierung von Erkrankungen, insbesondere Herz-Kreislauferkrankungen, Herzinsuffizienz, Infektionen und / oder Entzündungen von Lunge und Atemwege, sowie insbesondere unter Berücksichtigung der oben genannten Ausführungsformen. Die Markerkombination kann ggfs. einen weiteren geeigneten Marker enthalten.

Eine weitere Aufgabe ist die Bereitstellung einer entsprechenden diagnostischen Vorrichtung oder die Verwendung einer solchen Vorrichtung zur Durchführung des erfindungsgemäßen Verfahrens.

Im Rahmen dieser Erfindung wird unter einer solchen diagnostischen Vorrichtung, insbesondere ein Array oder Assay verstanden (z.B. Immunoassay, ELISA etc.), im weitesten Sinne eine Vorrichtung zur Durchführung des erfindungsgemäßen Verfahrens.

Offenbart ist ein Kit oder die Verwendung eines solchen Kit zur in-vitro Diagnose und Risikostratifizierung von Erkrankungen, insbesondere Herz-Kreislauferkrankungen, Herzinsuffizienz, Infektionen und / oder Entzündungen von Lunge und Atemwege, wobei eine Bestimmung von CT-proADM (SEQ ID No. 1) oder enthaltend in einer Markerkombination (Panel, Cluster) an einem zu untersuchenden Patienten durchgeführt wird, insbesondere unter Berücksichtigung der oben genannten Ausführungsformen. Solche Nachweisreagenzien umfassen z.B. Antikörper, etc.

Offenbart sind Antikörper, insbesondere monoklonale Antikörper zur Detektion von CT-proADM, die CT-proADM, vorzugsweise an den Bindungsstellen 1 - 15 oder 19-33 der SEQ ID. No. 1 jeweils unabhängig voneinander einzeln bindet oder an eines von beiden Bindungsstellen bindet oder an beiden Bindungsstellen binden.

Nachfolgende Beispiele und Figuren dienen zur näheren Erläuterung der Erfindung, jedoch ohne die Erfindung auf diese Beispiele und Figuren zu beschränken.

### Beispiele:

### Immunoassay

### Peptidsynthesen

Abgeleitet von der bekannten Aminosäuresequenz von preproADM wurden zwei Bereiche ausgewählt (Pos. 153-167, 171-185). Die Bereiche wurden, jeweils ergänzt um einen N-terminalen Cystein-Rest, nach Standardverfahren als lösliche Peptide chemisch synthetisiert, gereinigt, mittels Massenspektrometrie und Reversed Phase HPLC qualitätskontrolliert und in Aliquots lyophilisiert (Firma JPT, Berlin, Deutschland). Die Aminosäuresequenzen der Peptide lauten:
PSK16 CSLPEAGPGRTLVSSK Pos. 153-167
PHL16 CHGAPAPPSGSAPHFL Pos. 171-185
Desweiteren wurde ein Peptid synthetisiert, das den Bereich Pos. 153-185 von preproADM abdeckt (PSL33 SLPEAGPGRTLVSSKPQAFHGAPAPPSGSAPHFL).

### Konjugation und Immunisierung

Mittels MBS (m-Maleimidobenzoyl-N-hydroxysuccinimid Ester) wurden die Peptide PSK16 und PHL16 an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### Reinigung der Antikörper

In einem 1-Schritt Verfahren wurden aus den Antiseren, die beginnend mit dem vierten Monat nach der Immunisierung gewonnen worden waren, die Peptid-spezifischen Antikörper präpariert.
Hierzu wurden zunächst die Peptide PSK16 und PHL16 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen die Peptide wurde wie folgt durchgeführt:
Die Peptid-Säulen wurden zunächst dreimal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt. Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen à 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt. Nach Proteinbestimmung der Pools mittels der BCA-Methode ergaben sich Ausbeuten von 34 mg für den anti-PSK16 Antikörper und 48 mg für den anti- PHL16 Antikörper.

### Markierung

Über NAP-5 Gelfiltrationssäulen (Pharmacia) wurden 500 µl des gereinigten anti-PSK16 Antikörpers (s.o.) in 1 ml 100 mM Kalium-Phosphatpuffer (pH 8,0) nach Arbeitsanleitung umgepuffert. Die Proteinkonzentationen der Antikörperlösung wurden mit 100 mM Kalium-Phosphatpuffer (pH 8,0) auf 1,5 mg/ml eingestellt.

Zur Chemilumineszenzmarkierung wurde der Antikörper wie folgt weiterbehandelt: 67 µl der Antikörperlösung wurden mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsanastz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Duchflußphotometer wurden die Wellenlängen 280 nm und 368 nm gemessen. Das Absorbtionsverhältnis 368 nm/280 nm als Maß für den Markierungsgrad des Antikörpers betrug am Peak 0.10 +/- 0.01. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### Kopplung

Bestrahlte 5 ml Polystyrolröhrchen (Firma Greiner) wurden mit gereinigtem anti-PHL16 Antikörper wie folgt beschichtet: Der Antikörper wurde in 50 mM Tris, 100 mM NaCl, pH 7.8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### Durchführung und Auswertung des Immunoassays

Als Standardmaterial diente Peptid PSL33, das seriell in Pferdenormalserum (Firma SIGMA) verdünnt wurde. Den so hergestellten Standards wurden Konzentrationen gemäß der Peptideinwaage zugeschrieben.
Der Sandwichimmunoassay wurde wie folgt angesetzt: In die jeweiligen mit Antikörpern beschichteten Teströhrchen wurden 50 µl Standards bzw. Proben sowie 200 µl Assaypuffer (100 mM Natriumphosphat, 150 mM NaCl, 5% Bovines Serum Albumin, 0.1% unspez. Schaf IgG, 0.1% Natrium Azid, pH 7.4), enthaltend 1 Million RLU (relative light units) des MA70-markierten Antikörpers, pipettiert. Es wurde 2 Stunden bei 22°C unter Schütteln inkubiert. Dann wurde 4-mal mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen. Unter Verwendung der Software MultiCalc (Spline Fit) wurden die CT-proADM Konzentrationen der Proben an der Standardkurve abgelesen.

Analyt, der mit dem beschriebenen Assay gemessen werden kann, wird bezeichnet als C-terminales proAdrenomedullin (CT-proADM).

### Klinische Wertigkeit

### Normalbereich

CT-proADM Konzentrationen wurden in Proben von gesunden Kontrollpersonen (n=200) bestimmt. Der Median lag bei 77,6 pmol/L, der kleinste gemessene Wert bei 46,6, der größte bei 136,2 pmol/L, die 95% Percentilen bei 58,6 bzw. 113,8 pmol/L.

### Herzinsuffizienz / Schweregrad

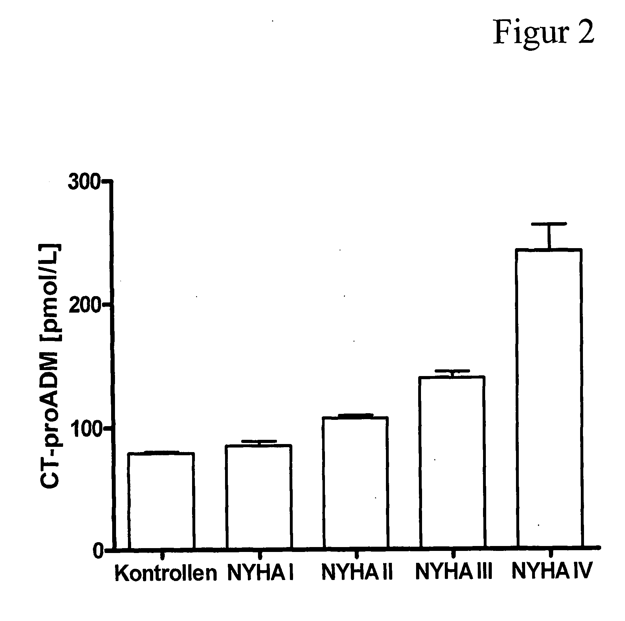

Es wurden CT-proADM Konzentrationen bei Patienten mit chronischer oder akut dekompensierter Herzinsuffizienz vermessen. CT-proADM Konzentrationen waren mit dem Schweregrad der Herzinsuffizienz assoziiert: Die Mittelwerte der CT-proADM Konzentrationen bei den vier Schweregrad-Kategorien NYHA I-IV waren: 85, 107, 140,4 bzw. 242,7 pmol/L (s. Fig. 2).

### Chronische Herzinsuffizienz / Diagnose

Von einem Kollektiv von 316 Patienten mit chronischer Herzinsuffizienz sowie 200 gesunden Kontrollen wurden CT-proADM Werte bestimmt. Die Receiver-Operator-Characteristics Analyse ergab eine AUC von 0,79. Bei einem Cut-off-Wert von 122 pmol/L ergab sich eine Sensitivität von 39,7% bei einer Spezifität von 98%. Bei einem Cut-off-Wert von 113 pmol/L ergab sich eine Sensitivität von 46,3% bei einer Spezifität von 95%.

### Chronische Herzinsuffizienz / Prognose

Von einem Kollektiv von 316 Patienten mit chronischer Herzinsuffizienz wurden CT-proADM Werte bestimmt. Die Patienten wurden über einen mittleren Zeitraum von 360 Tagen beobachtet. In diesem Zeitraum starben 42 Patienten, 274 überlebten. Durch Receiver-Operator-Characteristics Analyse wurde der beste Cut-off-Wert (definiert als das größte Produkt aus Sensitivität und Spezifität) zur Prognose der Mortalität ermittelt: 119.7 pmol/L. Bei diesem Cut-off-Wert betrug die Sensitivität der Prognose 73,2%, die Spezifität betrug 62,2%. Die Likelihood Ratio zu versterben betrug bei einem Cut-off-Wert von 119,7 pg/ml: 1.9.

| | <119,7 pmol/L | >119,7 pmol/L |
|---|---|---|
| Überlebende | 172 | 103 |
| Tote | 11 | 30 |

### Akute Herzinsuffizienz / Diagnose

Von einem Kollektiv von 125 Patienten mit akuter Atemnot wurden CT-proADM Werte bestimmt. Von den 125 Patienten hatten 69 Patienten Herzinsuffizienz. Die Receiver-Operator-Characteristics Analyse für die Differentialdiagnose der Herzinsuffizienz ergab eine AUC von 0,75. Bei einem Cut-off-Wert von 410 pmol/L ergab sich eine Sensitivität von 12,4% bei einer Spezifität von 98%. Bei einem Cut-off-Wert von 315 pmol/L ergab sich eine Sensitivität von 18,3% bei einer Spezifität von 95%.

### Akute Herzinsuffizienz / Prognose

Von einem Kollektiv von 69 Patienten mit akut dekompensierter Herzinsuffizienz wurden CT-proADM Werte bestimmt. Die Patienten wurden über einen Zeitraum von 360 Tagen beobachtet. In diesem Zeitraum starben 21 Patienten, 48 überlebten. Durch Receiver-Operator-Characteristics Analyse wurde der beste Cut-off-Wert (definiert als das größte Produkt aus Sensitivität und Spezifität) zur Prognose der Mortalität ermittelt: 192 pmol/L. Bei diesem Cut-off-Wert betrug die Sensitivität der Prognose, 66,6%, die Spezifität betrug 75%. Die Likelihood Ratio der Mortalität betrug bei einem Cut-off-Wert von 192 pmol/L: 2,5.

| | <192 pmol/L | >192 pmol/L, |
|---|---|---|
| Überlebende | 36 | 12 |
| Tote | 7 | 14 |

### Myokardinfarkt / Prognose

Von 287 Patienten mit akutem Herzinfarkt wurden drei Tage nach Eintreten des Herzinfarkts Proben gewonnen und es wurde CT-proADM gemessen. Die Patienten wurden über einen Zeitraum von 360 Tagen beobachtet. In diesem Zeitraum hatten 220 Patienten kein adverses Ereignis, 67 starben oder wurden wegen Herzinsuffizizienz rehospitalisiert. Durch Receiver-Operator-Characteristics Analyse wurde der beste Cut-off-Wert (definiert als das größte Produkt aus Sensitivität und Spezifität) zur Prognose der Mortalität bzw. Rehospitalisierung wegen Herzinsuffizienz ermittelt: 161,9 pmol/L. Bei diesem Cut-off-Wert betrug die Sensitivität der Prognose 67,2%, die Spezifität betrug 79,1%. Die Likelihood Ratio eines adversen Ereignisses betrug bei einem Cut-off-Wert von 161,9 pmol/L: 3.2.

| | <161,9 pmol/L | >161,9 pmol/L |
|---|---|---|
| Keine adverses Ereignis | 174 | 46 |
| Tot/Herzinsuffizienz | 22 | 45 |

### Pneumonie / Schweregrad und Prognose

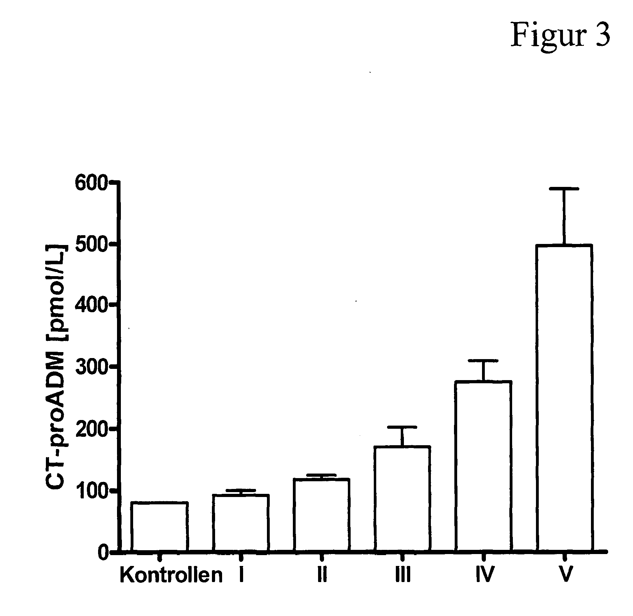

Von 142 Patienten mit ambulant erworbener Pneumonie wurden bei Aufnahme ins Krankenhaus Proben gewonnen und es wurde CT-proADM gemessen. Die Patienten wurden über einen Zeitraum von 70 Tagen beobachtet. In diesem Zeitraum starben 10 Patienten. CT-proADM Konzentrationen stiegen mit dem PSI (Pneumonia Severity Index), einem Score für den Schweregrad der Erkrankung (Fig. 3) und waren im Median mit 135 pmol/L gegenüber gesunden Kontrollen (77 pmol/L) erhöht. Für die Prognose der Mortalität erbrachte die Receiver-Operator-Characteristics Analyse eine AUC von 0,89. Durch Receiver-Operator-Characteristics Analyse wurde der beste Cut-off-Wert (definiert als das größte Produkt aus Sensitivität und Spezifität) zur Prognose der Mortalität ermittelt: 194,5 pmol/L. Bei diesem Cut-off-Wert betrug die Sensitivität der Prognose 100%, die Spezifität betrug 81,2%. Die Likelihood Ratio der Mortalität betrug bei einem Cut-off-Wert von 194,5 pmol/L: 5.5.

| | <194,5 pmol/L | >194,5 pmol/L |
|---|---|---|
| Keine adverses Ereignis | 108 | 24 |
| Tot | 0 | 10 |

### Exacerbierte COPD

Von 53 Patienten mit chronisch obstruktiver Lungenerkrankung und gleichzeitiger Infektion der unteren Atemwege wurde CT-proADM gemessen. Mit im Median 106 pmol/L waren diese Patienten gegenüber gesunden Kontrollen (77 pmol/L) erhöht, lagen jedoch niedriger als Pneumonie Patienten (s.o. 135 pmol/L).

## Patentansprüche

1. Verfahren zur in-vitro Diagnose und/oder Risikostratifizierung von Erkrankungen,
**dadurch gekennzeichnet,**
**dass** eine Bestimmung von dem C-terminalen Fragment von proAdrenomedullin (CT-proADM) (SEQ ID No. 1) von einem zu untersuchenden Patienten durchgeführt wird.

2. Verfahren nach Anspruch 1, **dadurch gekennzeichnet, dass** die in-vitro Diagnose und / oder Risikostratifierung von Erkrankungen, insbesondere von Herz-Kreislauferkrankungen, Herzinsuffizienz, Infektionen und / oder Entzündungen von Lunge und Atemwege erfolgt.

3. Verfahren nach Anspruch 2,
**dadurch gekennzeichnet,**
**dass** die Herz-Kreislauferkrankungen Bluthochdruck, koronare Herzerkrankungen, insbesondere akutes Koronarsyndrom, (akuter) Myokardinfarkt, Angina Pectoris umfassen.

4. Verfahren nach Anspruch 2 oder 3,
**dadurch gekennzeichnet,**
**dass** die Herzinsuffizienz eine chronische Herzinsuffizienz, hypertensive Herzkrankheit mit (kongestiver) Herzinsuffizienz, hypertensive Herz- und Nierenkrankheit mit (kongestiver) Herzinsuffizienz, Primäre Rechtsherzinsuffizienz, Sekundäre Rechtsherzinsuffizienz, Linksherzinsuffizienz ohne Beschwerden (NYHA-Stadium I), Linksherzinsuffizienz mit Beschwerden bei stärkerer Belastung (NYHA-Stadium II), Linksherzinsuffizienz mit Beschwerden bei leichterer Belastung (NYHA-Stadium III), Linksherzinsuffizienz mit Beschwerden in Ruhe (NYHA-Stadium IV), kardiogener Schock, Myokarditis, Herzrhythmusstörungen und / oder Hypertonie umfasst.

5. Verfahren nach Anspruch 2,
**dadurch gekennzeichnet,**
**dass** die Infektionen und / oder Entzündungen von Lunge und Atemwege
a.) Infektionen, die durch Bakterien, Viren, Pilze oder Parasiten verursacht werden, tiefe Atemwegsinfektion (LRTI: Lower respiratory tract infections), Bronchitis, Pneumonie, Sarkoidose, Bronchiektasen, Nicht-kardiale Lungenödem und / oder
b.) Interstitielle Lungenerkrankungen und Lungenfibrosen, Chronisch obstruktive Lungenerkrankungen (COPD) insbesondere COPD Infekt-Exazerbationen, Asthma bronchiale, insbesondere Infekt-Exazerbationen bei Asthma brcnchiale, Bronchialkarzinom umfassen.

6. Verfahren nach einem der vorherigen Ansprüche,
**dadurch gekennzeichnet,**
**dass** zusätzlich eine Bestimmung mindestens eines weiteren Markers ausgewählt aus der Gruppe inflammatorischer Marker, und zwar mindestens einem Marker aus der Gruppe C-reaktives Protein (CRP) Cytokinen, wie etwa TNF-alpha, Interleukinen, wie etwa IL-6, Procalcitonin (1-116, 3-116) und Adhäsionsmolekülen, wie VCAM oder ICAM ausgewählt ist, cardiovaskulärer Marker, und zwar mindestens einem Marker aus der Gruppe Kreatinkinase, Myeloperoxidase, Copeptin, Myoglobin, natriuretisches Protein, insbesondere ANP (bzw. ANF), proANP, NT-proANP, BNP, proBNP, NT-proBNP oder jeweils eine Teilsequenz davon, kardiale Troponin, CRP sowie Kreislaufregulierende (Pro)Hormone, wie pro-Gastrin-Releasing Peptid (proGRP), pro-Endothelin-1, pro-Leptin, pro-Neuropeptid-γ, pro-Somatostatin, pro-Neuropeptid-YY, pro-Opiomelanocortin, Copeptin oder jeweils eine Teilsequenz davon ausgewählt ist, ischämischer Marker, und zwar mindestens einem Marker aus der Gruppe Troponin I und T, CK-MB ausgewählt ist, oder neurohormonaler Marker, und zwar mindestens einem Marker aus der Gruppe der natriuretischen Proteine ist, insbesondere ANP (bzw. ANF), proANP, NT-proANP, BNP, proBNP, NT-proBNP oder eine Teilsequenz davon ist, von einem zu untersuchenden Patienten durchgeführt wird.

7. Verfahren nach einem der vorherigen Ansprüche,
**dadurch gekennzeichnet,**
**dass** parallele oder simultane Bestimmungen der Marker durchgeführt werden.

8. Verfahren nach einem der vorherigen Ansprüche,
**dadurch gekennzeichnet,**
**dass** die Bestimmungen an mindestens einer Patientenprobe durchgeführt werden.

9. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

10. Verfahren nach einem der vorherigen Ansprüche, **dadurch gekennzeichnet, dass** die Diagnose zur Stratifizierung von Patienten für klinische Entscheidungen, insbesondere weiterführende Behandlung mittels Arzneimitteln zur Behandlung oder Therapie von Erkrankungen, insbesondere in der Intensivmedizin oder Notfallmedizin und zur Hospitalisierung des Patienten erfolgt.

11. Verfahren nach einem der vorheriger. Ansprüche, **dadurch gekennzeichnet, dass** die Diagnose und / oder Risikostratifizierung zur Prognose, zur differentialdiagnostischen Früherkennung und Erkennung, zur Beurteilung des Schweregrades und zur therapiebegleitenden Verlaufsbeurteilung von Erkrankungen, insbesondere von Herz-Kreislauferkrankungen, Herzinsuffizienz, Infektionen und / oder Entzündungen von Lunge und Atemwege erfolgt.

12. Verwendung von CT-proADM (SEQ ID No. 1) zur in-vitro Diagnose und /oder Risikostratifizierung von Erkrankungen, insbesondere von Herz-Kreislauferkrankungen, Herzinsuffizienz, Infektionen und / oder Entzündungen von Lunge und Atemwege.

13. Verwendung von CT-proADM (SEQ ID No. 1) zur *in vitro* Kardialdiagnostik.

14. Verwendung von CT-proADM (SEQ ID No. 1) oder enthaltend in einer Markerkombination zur in-vitro Diagnose und /oder Risikostratifizierung von Erkrankungen, insbesondere von Herz-Kreislauferkrankungen, Herzinsuffizienz, Infektionen und / oder Entzündungen von Lunge und Atemwege, wobei die Markerkombination weitere Marker nach Anspruch 6 enthält.

## Claims

1. Method for *in-vitro* diagnosis and/or stratifying the risk of diseases,
**characterized in that**
the C-terminal fragment of proAdrenomedullin (CT-proADM) (SEQ ID No. 1) is determined from a patient who is to be examined.

2. Method according to claim 1,
**characterized in that** the *in-vitro* diagnosis or risk stratification of diseases, particularly cardiovascular diseases, cardiac insufficiency, infections and / or inflammations of the lungs and respiratory tract is determined.

3. Method according to claim 2,
**characterized in that**
the cardiac diseases comprise high blood pressure, coronary heart diseases, especially acute coronary syndrome, (acute) myocardial infarct, angina pectoris.

4. Method according to claim 2 or claim 3,
**characterized in that**
the cardiac insufficiency comprises a chronic cardiac insufficiency, hypertensive heart disease with (congestive) cardiac insufficiency, hypertensive heart and kidney disease with (congestive) cardiac insufficiency, primary right-ventricular heart failure, secondary right-ventricular heart failure, left-ventricular heart failure without discomfort (NYHA stage I), left-ventricular heart failure with discomfort under increased stress (NYHA stage II), left-ventricular heart failure with discomfort under light stress (NYHA stage III), left-ventricular heart failure with discomfort at rest (NYHA stage IV), cardiogenic shock, myocarditis, cardiac arrhythmia and / or hypertension.

5. Method according to claim 2
**characterized in that**
the infections and / or inflammations of the lungs and respiratory tract comprise
a.) infections caused by bacteria, viruses, fungi or parasites, deep respiratory infections (LRTI: Lower respiratory tract infections), bronchitis, pneumonia, sarcoidosis, bronchiectasias, non-cardiac pulmonary edema and / or
b.) interstitial lung diseases and lung fibroses, chronic obstructive pulmonary diseases (COPD), particularly COPD infection exacerbations, bronchial asthma, in particular infection exacerbations with bronchial asthma, bronchial carcinoma.

6. Method according to one of the previous claims
**characterized in that**
additionally at least one additional marker - selected from a group of inflammatory markers namely at least one marker selected from the group of C-reactive protein (CRP), cytokines, such as TNF-alpha, interleukins, such as IL-6, procalcitonin (1-116, 3-116) and adhesion molecules, such as VCAM or ICAM,
cardiovascular markers namely at least one marker from the group of creatine kinase, kinase, myeloperoxidase, copeptin, myoglobin, natriuretic protein, especially ANP (or ANF), proANP, NT-proANP, BNP, proBNP, NT-proBNP, or in each case a partial sequence thereof, cardiac troponin, CRP, as well as (pro)hormones regulating circulation, such as pro-gastrin-releasing peptide (proGRP), pro-endothelin-1, pro-leptin, pro-neuropeptide-Y, pro-somatostatin, pro-neuropeptide-YY, pro-opiomelanocortin, copeptin or in each case a partial sequence thereof,
ischemic markers namely at least one marker from the group of troponin I and T, CB-MB,
neurohormonal marker namely at least a natriuretic protein, particularly ANP (or ANF), proANP, NT-proANP, BNP, proBNP, NT-proBNP, or in each case a partial sequence thereof
- is determined from a patient who is to be examined.

7. Method according to one of the previous claims
**characterized in that**
parallel or simultaneous determinations of the markers are carried out.

8. Method according to one of the previous claims
**characterized in that**
the determinations are carried out by means of at least one patient sample.

9. Method according to one of the previous claims **characterized in that** the determinations carried out by means of a rapid test, particularly through individual or multi-parameter determinations.

10. Method according to one of the previous claims **characterized in that** the diagnosis for the stratification of patients, in particular for the stratification of patients is made for clinical decisions, particularly further treatment by means of using drugs for the treatment or therapy of diseases, especially in intensive medicine or emergency medicine and for the hospitalization of patients.

11. Method according to one of the previous claims **characterized in that** the diagnosis and / or risk stratification is made for prognosis, for differential-diagnostic early diagnosis and detection, for the assessment of the severity, and for the evaluation of the course of diseases accompanying the therapy, particularly cardiovascular diseases, cardiac insufficiency, infections and / or inflammations of the lungs and respiratory tract.

12. Use of CT-proADM (SEQ ID No. 1) for the *in-vitro* diagnosis and / or risk stratification of diseases, particularly cardiovascular diseases, cardiac insufficiency, infections and / or inflammations of the lungs and respiratory tract.

13. Use of CT-proADM (SEQ ID No. 1) for *in-vitro* cardiac diagnostics.

14. Use of CT-proADM (SEQ ID No. 1), or contained in a marker combination for the *in-vitro* diagnosis and / or risk stratification of diseases, particularly cardiovascular diseases, cardiac insufficiency, infections and / or inflammations of the lungs and respiratory tract, said marker combination containing additional markers according to claim 6.

## Revendications

1. Méthode de diagnostic *in vitro* et/ou de stratification du risque de maladies, **caractérisée par le fait que** le fragment C-terminal de la proAdrénomédulline (CT-proADM) (SEQ ID No. 1) est déterminé à partir d'un patient qui doit être examiné.

2. Méthode selon la revendication 1, **caractérisée par le fait que** le diagnostic *in vitro* ou la stratification du risque de maladies, en particulier de maladies cardiovasculaires, d'une insuffisance cardiaque, d' infections et/ou inflammations des poumons et du tractus respiratoire, est déterminé.

3. Méthode selon la revendication 2, **caractérisée par le fait que** les maladies cardiaques comprennent l'hypertension artérielle, les coronaropathies, en particulier le syndrome coronarien aigu, l'infarctus du myocarde (aigu), l'angine de poitrine.

4. Méthode selon l'une des revendications 2 ou 3, **caractérisée par le fait que** l'insuffisance cardiaque comprend une insuffisance cardiaque chronique, une cardiopathie hypertensive avec une insuffisance cardiaque (congestive), une cardiopathie et néphropathie hypertensives avec une insuffisance cardiaque (congestive), une insuffisance cardiaque primaire du ventricule droit, une insuffisance cardiaque secondaire du ventricule droit, une insuffisance cardiaque du ventricule gauche sans gêne (NYHA stade I), une insuffisance cardiaque du ventricule gauche avec gêne dans des conditions de stress accru (NYHA stade II), une insuffisance cardiaque du ventricule gauche avec gêne dans des conditions de stress léger (NYHA stade III), une insuffisance cardiaque du ventricule gauche avec gêne au repos (NYHA stade IV), un choc cardiogène, une myocardite, une arythmie cardiaque et/ou une hypertension.

5. Méthode selon la revendication 2, **caractérisée par le fait que** les infections et/ou inflammations des poumons et du tractus respiratoire comprennent
a.) des infections provoquées par des bactéries, des virus, des champignons ou des parasites, des infections respiratoires profondes (LRTI : infections du tractus respiratoire inférieur), bronchite, pneumonie, sarcoïdose, bronchiectasies, un oedème pulmonaire non cardiaque et/ou
b.) des maladies pulmonaires interstitielles et des fibroses pulmonaires, des maladies pulmonaires obstructives chroniques (MPOC), en particulier des exacerbations d'une infection MPOC, l'asthme, en particulier des exacerbations d'infection avec de l'asthme, un carcinome bronchial.

6. Méthode selon l'une des revendications précédentes, **caractérisée par le fait qu'**en plus, au moins un marqueur supplémentaire - choisi dans un groupe constitué de :
marqueurs inflammatoires, à savoir au moins un marqueur choisi dans le groupe consistant en la protéine C-réactive (CRP), des cytokines, telles que TNF-alpha, des interleukines, telles que IL-6, la procalcitonine (1-116, 3-116) et des molécules d'adhésion, telles que VCAM ou ICAM,
marqueurs cardiovasculaires, à savoir au moins un marqueur parmi le groupe de la créatine kinase, une kinase, la myélopéroxydase, la copeptine, la myoglobine, une protéine natriurétique, en particulier ANP (ou ANF), proANP, NT-proANP, BNP, proBNP, NT-proBNP ou, dans chacun des cas, une séquence partielle de ceux-ci, la troponine cardiaque, CRP, ainsi que des (pro)hormones régulant la circulation, telles que le peptide de libération de la pro-gastrine (proGRP), la pro-endothéline-1, la pro-leptine, le pro-neuropeptide-Y, la pro-somatostatine, le pro-neuropeptide-YY, la pro-opiomélanocortine, la copeptine ou, dans chacun des cas, une séquence partielle de ceux-ci,
marqueurs ischémiques, à savoir au moins un marqueur du groupe de la troponine I et T, CB-MB,
marqueur neurohormonal, à savoir au moins une protéine natriurétique, en particulier ANP (ou ANF), proANP, NT-proANP, BNP, proBNP, NT-proBNP ou, dans chacun des cas, une séquence partielle de ceux-ci
- est déterminé à partir d'un patient qui doit être examiné.

7. Méthode selon l'une des revendications précédentes, **caractérisée par le fait que** des déterminations parallèles ou simultanées des marqueurs sont mises en oeuvre.

8. Méthode selon l'une des revendications précédentes, **caractérisée par le fait que** les déterminations sont mises en oeuvre au moyen d'au moins un échantillon de patient.

9. Méthode selon l'une des revendications précédentes, **caractérisée par le fait que** les déterminations sont mises en oeuvre au moyen d'un test rapide, en particulier par des déterminations individuelles ou à multiples paramètres.

10. Méthode selon l'une des revendications précédentes, **caractérisée par le fait que** le diagnostic pour la stratification des patients, en particulier pour la stratification de patients, est réalisé pour des décisions cliniques, en particulier pour un traitement ultérieur par l'utilisation de médicaments pour le traitement ou la thérapie de maladies, en particulier en soins intensifs ou en médecine d'urgence et pour l'hospitalisation de patients.

11. Méthode selon l'une des revendications précédentes, **caractérisée par le fait que** le diagnostic et/ou la stratification du risque est réalisé pour le pronostic, pour le diagnostic et la détection précoces par diagnostic différentiel, pour l'estimation de la sévérité et pour l'évaluation du déroulement des maladies accompagnant la thérapie, en particulier de maladies cardiovasculaires, d'une insuffisance cardiaque, d'infections et/ou inflammations des poumons et du tractus respiratoire.

12. Utilisation de CT-proADM (SEQ ID No. 1) pour le diagnostic *in vitro* et/ou la stratification du risque de maladies, en particulier de maladies cardiovasculaires, d'une insuffisance cardiaque, d'infections et/ou inflammations des poumons et du tractus respiratoire.

13. Utilisation de CT-proADM (SEQ ID No. 1) pour des diagnostics cardiaques *in vitro.*

14. Utilisation de CT-proADM (SEQ ID No. 1), ou CT-proADM contenu dans une combinaison de marqueurs pour le diagnostic *in vitro* et/ou la stratification du risque de maladies, en particulier des maladies cardiovasculaires, une insuffisance cardiaque, des infections et/ou inflammations des poumons et du tractus respiratoire, ladite combinaison de marqueurs contenant des marqueurs supplémentaires selon la revendication 6.


### SEQUENCE LISTING

<110> Brahms Aktiengesellschaft
<120> Diagnose und Risikostratifizierung mittels dem neuen Marker CT-proADM
<130> 06/15 BRAHMS 32WO
<140> 10 2006 060 112.2
   <141> 2006-12-20
<160> 4
<170> PatentIn version 3.3
<210> 1
   <211> 33
   <212> PRT
   <213> Human
<400> 1
   Ser Leu Pro Glu Ala Gly Pro Gly Arg Thr Leu Val Ser Ser Lys Pro
   1               5                   10                  15
   Gln Ala His Gly Ala Pro Ala Pro Pro Ser Gly Ser Ala Pro His Phe
               20                  25                  30
   Leu
<210> 2
   <211> 185
   <212> PRT
   <213> Human
<400> 2
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Arg Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 3
   <211> 16
   <212> PRT
   <213> Human
<400> 3
   Cys Ser Leu Pro Glu Ala Gly Pro Gly Arg Thr Leu Val Ser Ser Lys
   1               5                   10                  15
<210> 4
   <211> 16
   <212> PRT
   <213> Human
<400> 4
   Cys His Gly Ala Pro Ala Pro Pro Ser Gly Ser Ala Pro His Phe Leu
   1               5                   10                  15